# EUROPEAN PATENT APPLICATION

(11) **EP 3 822 983 A1**
(43) Date of publication of application: **19.05.2021**
(21) Application number: 19209816.8
(22) Date of filing: 18.11.2019
(51) Int. Cl.: G16H 30/20, G16H 30/40, G16H 40/63, G16H 50/20

(54) **LEARNING, INDEXING, AND/OR APPLYING ASPECTS OF DIGITAL HANGING PROTOCOLS**

(71) Applicant: Koninklijke Philips N.V., 5656 AG Eindhoven (NL)
(72) Inventor: BERGTHOLDT, Martin, 5656 AE Eindhoven (NL); KLINDER, Tobias, 5656 AE Eindhoven (NL); WIEMKER, Rafael, 5656 AE Eindhoven (NL); LENGA, Matthias, 5656 AE Eindhoven (NL); CAROLUS, Heike, 5656 AE Eindhoven (NL)
(74) Representative: Philips Intellectual Property & Standards

(57) **Abstract**

Embodiments are described herein for learning, indexing, and/or applying digital hanging protocols. In various embodiments, a clinical task to be performed with a medical examination software application (104) in association with medical imagery data acquired from a subject may be identified (702). The medical imagery data may be analyzed (704) to establish a frame of reference with respect to one or more anatomical features of the subject depicted in the medical imagery data. A digital hanging protocol associated with the clinical task may be retrieved (706). The digital hanging protocol may include a plurality of predefined views learned from previous performances of the clinical task. One of the predefined views may be selected (708), and display data that includes a portion of the medical imagery data that is identified based on the selected view and the established frame of reference may be generated (712) for rendition on a display.

## Description

### FIELD OF THE INVENTION

Various embodiments described herein are directed generally to health care. More particularly, but not exclusively, various methods and apparatus disclosed herein relate to learning, indexing, and/or applying aspects of digital hanging protocols.

### BACKGROUND OF THE INVENTION

Medical imagery data takes various forms, including but not limited to computerized tomography ("CT") scans, magnetic resonance imaging ("MRI"), positron emission tomography ("PET") scans, single photon emission computed tomography ("SPECT") scans, three-dimensional ("3D") ultrasounds, 3D cine-loops, and contrast enhanced dynamic MRI, to name a few. With any of these forms of medical imagery, a user such as a radiologist or other medical personnel "navigates" slice-wise through the 3D medical imagery as part of his or her examination, in a process referred to herein as "medical imagery reading."

As medical imagery and workflows for performing medical imagery reading are becoming increasingly digital, the medical personnel's workload has also increased dramatically, especially with the larger fields of view and higher resolution medical imagery that is becoming more commonplace. Moreover, different medical personnel might have different workflows to perform medical imagery reading. There may also be hospital-wide, regional, and/or nationwide guidelines for various clinical tasks. Medical imagery reading also commonly involves a significant amount of user interactions besides the "slice scrolling" for navigation, including but not limited to zooming, panning, scrolling, rotating, change of displayed image orientation/reformat, and so forth.

### SUMMARY OF THE INVENTION

The present disclosure is directed to methods and apparatus for learning, indexing, and/or applying aspects of digital hanging protocols. For example, techniques are described herein for learning recurring viewport settings for various medical tasks based on user interactions with a medical diagnostic software application and/or workstation. By automatically analyzing medical imagery data using techniques such as anatomy localization and/or computer-aided detection for clinically relevant, suspicious, or pathologic findings, techniques described herein facilitate task-driven workflow guidance. Techniques described herein also address the problems of large data and limited bandwidth in the emerging field of tele-radiology.

As used herein, a "clinical task" may refer to a workflow or process performed by medical personnel such as a radiologist or pathologist with a particular purpose or purposes in mind. In the context of the present disclosure, a clinical task will involve a medical personnel operating a medical examination software application to view, read, examine, annotate, and/or otherwise interact with medical imagery data, *e.g.,* to identify one or more lesions or other abnormalities in a subject.

The term "lesion" as used herein may refer to a region in an organ or tissue which has suffered damage through injury or disease, such as a wound, ulcer, abscess, tumor, aneurysm, fracture (*e.g.,* in bone), tear (*e.g.,* in a tendon or ligament), etc. The term "lesion" may also refer to inflammation, air or liquid buildup, and/or any other abnormal increase or decrease in size of a particular anatomical feature (*e.g.,* enlarged lymph nodes). Clinical tasks may take myriad forms because there are innumerable scenarios in which medical personnel may be tasked with reading and/or examining medical imagery data to detect lesions, make diagnoses, form treatment plans, plan surgeries, and so forth.

One broad category of clinical tasks is evaluation of trauma events in which subjects experience physical trauma that may or may not require diagnosis and/or treatment. Non-limiting examples of trauma events include car accidents, sports accidents, workplace accidents, assaults, or any other scenario or occurrence that resulted in bodily harm to a subject. Lesions that may be associated with trauma events include tears, fractures, strains, breaks, etc. Another broad category of clinical tasks is evaluation of a disease or condition that is suspected based on various factors, such as symptoms, demographics, etc. Some diseases or conditions may be exogenous (*e.g.,* caused by pollution, poor nutrition, and/or other external factors) and other diseases or conditions may be endogenous (*e.g.,* genetic disorders, some chronic conditions such as diabetes). Lesions that may be associated with diseases or conditions include, but are not limited to, tumors, abscesses, aneurysms, polyps, bone spurs, joint damage, damage to cartilage (*e.g.,* spinal disks), and so forth.

As used herein, a "hanging protocol" refers to series of actions, or a workflow, performed to arrange printed medical images for optimal viewing, *e.g.,* by medical personnel such as radiologists. Particular clinical tasks may call for a particular hanging protocol. For example, when reading a trauma scan acquired in response to a suspected spinal injury, radiologists may prefer to be presented with a particular set of views of the subject's spine from a particular set of angles, so that the radiologists can more quickly diagnose any spinal injury that may have occurred. In some cases, technicians such as X-ray, ultrasound, MRI, or CT technicians may perform the actions designated in a hanging protocol to ensure the clinician is presented with the proper medical images. "Digital" hanging protocols are similar to physical hanging protocols, except they refer to actions performed to generate a particular set or sequence of digital images obtained from digital imagery acquired from the subject.

Generally, in one aspect, a method may include: identifying a clinical task to be performed with a medical examination software application in association with medical imagery data acquired from a subject; analyzing the medical imagery data to establish a frame of reference with respect to one or more anatomical features of the subject depicted in the medical imagery data; retrieving a digital hanging protocol associated with the clinical task, wherein the digital hanging protocol includes a plurality of predefined views learned from previous performances of the clinical task; selecting one of the predefined views; and generating, for rendition on a display, display data that includes a portion of the medical imagery data that is identified based on the selected view and the established frame of reference.

In various embodiments, the plurality of predefined views may be indexed by corresponding measures of prior interest. In various embodiments, the corresponding measures of prior interest may be based on historical user interaction with the medical examination software application. In various embodiments, the historical user interaction with the medical examination software application may include time intervals spent by users interacting with the plurality of predefined views.

In various embodiments, the method may further include analyzing the medical imagery data to detect one or more lesions of the subject. In various embodiments, selecting one of the predefined views may include selecting one of the predefined views based on the detected one or more lesions. In various embodiments, the method may further include selecting the digital hanging protocol from a library of digital hanging protocols based on the detected one or more lesions.

In various embodiments, the medical imagery data may be stored on a server computer and the medical examination software application executes at least in part on a client computer in network communication with the server computer. In various embodiments, the method may further include proactively downloading and caching, by the client computer from the server computer, one or more portions of the medical imagery data that correspond to one or more unselected predefined views of the plurality of predefined views.

In various embodiments, the method may further include: generating, for rendition on the display, additional display data that includes one or more additional portions of the medical imagery data that are identified based on one or more additional predefined views and the established frame of reference; and automatically transitioning between rendering the display data corresponding to the selected predefined view and the additional display data corresponding to the one or more additional predefined views.

In another aspect, a method may include: receiving a plurality of user input sequences provided at a medical examination software application, wherein the medical examination software application facilitates user engagement with medical imagery data, wherein the plurality of user input sequences define a corresponding plurality of views of the medical imagery data, and wherein each of the plurality of views focuses the medical examination software application on a respective portion of the medical imagery data; determining, based on user interaction with the medical examination software application, measures of user interest corresponding to the plurality of views of the medical imagery data; and storing, in a database, the plurality of views in association with the measures of user interest.

In addition, some implementations include one or more processors of one or more computing devices, where the one or more processors are operable to execute instructions stored in associated memory, and where the instructions are configured to cause performance of any of the aforementioned methods. Some implementations also include one or more non-transitory computer readable storage media storing computer instructions executable by one or more processors to perform any of the aforementioned methods.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

### BRIEF DESCRIPTION OF THE DRAWINGS

In the drawings, like reference characters generally refer to the same parts throughout the different views. Also, the drawings are not necessarily to scale, emphasis instead generally being placed upon illustrating various principles of the embodiments described herein.
Fig. 1 illustrates an example environment in which selected aspects of the present disclosure may be implemented, in accordance with various embodiments.
Figs. 2A and 2B depict examples of how hanging protocols and/or views thereof may be selected, indexed, ranked, and/or presented, in accordance with various embodiments.
Fig. 3 depicts an example graphical user interface ("GUI") that may be presented to medical personal to perform medical imagery reading, and from which user interactions may be used to learn and/or improve digital hanging protocols using techniques described herein.
Fig. 4 depicts an example GUI that may be presented using techniques described herein.
Fig. 5 depicts another example GUI that may be presented using techniques described herein.
Fig. 6 depicts an example method for practicing selected aspects of the present disclosure.
Fig. 7 depicts an example method for practicing selected aspects of the present disclosure.
Fig. 8 depicts an example computer architecture.

### DETAILED DESCRIPTION OF EMBODIMENTS

As noted previously, as medical imagery data and medical imagery workflows become increasingly digitized and computing resources increase, medical imagery resolution and workloads of medical personnel such as radiologists are also increasing dramatically. In view of the foregoing, various embodiments and implementations of the present disclosure are directed to learning, indexing, and/or applying aspects of digital hanging protocols to increase the efficiency of, and/or reduce mistakes made by, medical personnel such as radiologists.

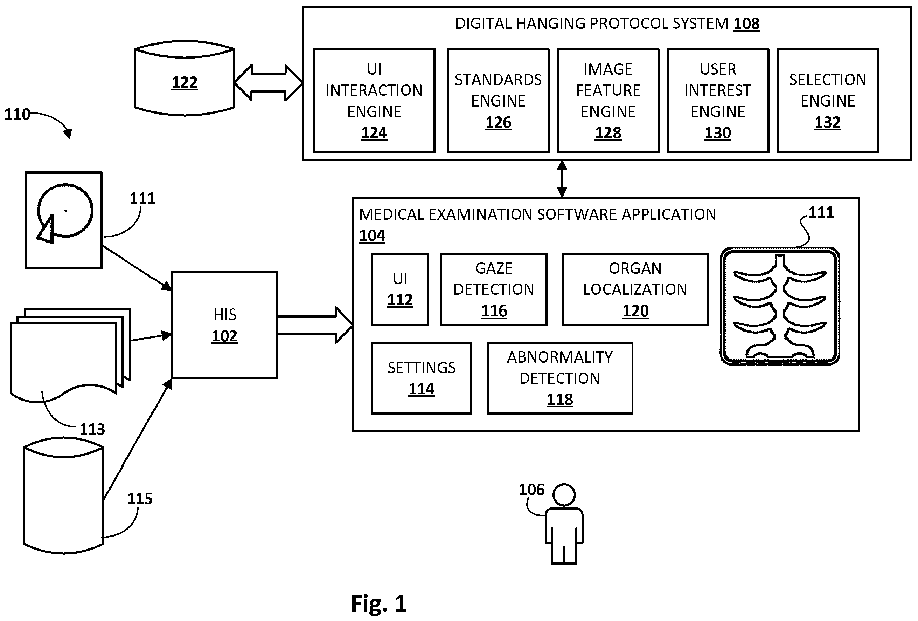

Fig. 1 schematically depicts an example environment in which selected aspects of the present disclosure may be implemented, in accordance with various embodiments. The computing devices depicted in Fig. 1 and elsewhere herein may include, for example, one or more of: a desktop computing device, a laptop computing device, a tablet computing device, a mobile phone computing device, a computing device of a vehicle of the user (*e.g.,* an in-vehicle communications system, an in-vehicle entertainment system, an in-vehicle navigation system), a standalone interactive speaker (which in some cases may include a vision sensor), a smart appliance such as a smart television (or a standard television equipped with a networked dongle with automated assistant capabilities), a server computing device (*e.g.,* local or cloud-based), and/or a wearable apparatus of the user that includes a computing device (*e.g.,* a watch of the user having a computing device, glasses of the user having a computing device, a virtual or augmented reality computing device). Additional and/or alternative computing devices may be provided.

A variety of data sources 110 are available from which a medical imagery data can be obtained. In this example, these data sources 110 include one or more computer hard drives 111 from which data can be extracted, for instance, from files, etc. Also present are one or more documents 113, and one or more databases 115 associated with various information systems. In the medical context, data sources 110 may include data sources like electronic medical records ("EMR"), a hospital information system ("HIS") 102, hospital equipment, etc., and other sources of medical imagery. In some medical contexts, medical imagery data may include computerized tomography ("CT") scans, magnetic resonance imaging ("MRI"), positron emission tomography ("PET") scans, single photon emission computed tomography ("SPECT") scans, three-dimensional ("3D") ultrasounds, 3D cine-loops, and contrast enhanced dynamic MRI, to name a few. These medical imagery data may be stored using various formats, including but not limited to the Picture Archiving and Communication System ("PACS"), the Digital Imaging and Communications in Medicine ("DICOM"), etc.

In some implementations, HIS 102 may be configured to retrieve and/or organize medical imagery data from data sources 110. HIS 102 may provide this retrieved/organized medical imagery data to a medical examination software application 104 operated by one or more medical personnel 106 (*e.g.,* a radiologist or pathologist). Medical examination software application 104 may present aspects and/or portions of the medical imagery data in manners that are determined, *e.g.,* by digital hanging protocol system 108, using techniques described herein. For example, medical examination software application 104 may present medical personnel 106 with one or more views 111 of portions of the medical imagery data that are selected using techniques described herein. Medical personnel 106 may then read and/or study the medical imagery data depicted in t these view(s) to, for instance, make diagnoses about subjects such as patients (not depicted), form treatment plans, plan surgeries, etc.

Medical examination software application 104 may include a variety of different components that serve various purposes and/or provide features that may or may not be utilized with techniques described herein. These components may be implemented using any combination of hardware and software. For example, in Fig. 1, medical examination software application 104 includes a user interface ("UI") module 112, a settings module 114, a gaze detection module 116, an abnormality detection module 118, and an organ localization module 120. One or more of these modules may be combined and/or omitted in other embodiments. Generally speaking, medical examination software application 104 may be executed on a single computer and/or across multiple computers, *e.g.,* as a client-server application.

UI module 112 may provide various user interface elements which receive input and/or provide output, *e.g.,* to medical personnel 106. These user interface elements may be visual, audible, and/or haptic/tactile. For example, UI module 112 may provide a graphical user interface ("GUI") on a display (not depicted) that medical personnel 106 can interact with, *e.g.,* using a mouse, keyboard, stylus, etc.-to perform medical examination (*e.g.,* medical imagery reading) in a manner that is streamlined using techniques described herein.

In some embodiments, UI module 112 may record and/or log information about user interaction with medical examination software application 104 during performance of a particular clinical task. This recorded user interaction information may be used, *e.g.,* by digital hanging protocol system 108, to create and/or improve a digital hanging protocol associated with a clinical task being performed by medical personnel 106. For example, in some embodiments, a measure of user interaction and/or interest with a particular view of medical imagery of a subject (*e.g.,* zoomed into a particular area of a particular organ) may be determined based on information provided by UI module 112. This information may include, for instance, an amount of user interaction with the view (*e.g.,* how much the user tweaked various settings or panned around the view), how long a particular view was active or in the foreground, and so forth. As described herein, this information may be used to generate and/or improve a digital hanging protocol associated with the clinical task being performed.

Settings module 114 may maintain, manage, and/or record settings implemented and/or selected by medical personnel 106 while operating medical examination software application 104 to read medical imagery. These settings may be selected in some embodiments using user interface elements provided by UI module 112. In some embodiments, modules 112 and 114 may be combined into a single module. Medical personnel 106 may select and/or implement a variety of different settings when reading medical imagery data. Some non-limiting examples are digital image processing settings such as color balance, hue, saturation, contrast, gamma levels, brightness/intensity, filters, or any other parameter or setting that is adjustable by medical personnel 106 to enable them to better detect and/or diagnose lesions. Settings that are recorded by settings module 114 maybe used, *e.g.,* by digital hanging protocol system 108, to create and/or improve a digital hanging protocol associated with a clinical task being performed by medical personnel 106.

Gaze detection module 116 is an optional module that may detect, *e.g.,* using a vision sensor (not depicted) such as a camera or infrared ("IR") sensor, various aspects of a gaze of medical personnel 106. If medical personnel 106 looks at a particular view of a particular portion of medical imagery for an exceptionally long time interval, that may be a signal that the view is relatively useful or important, and should be included and/or highly-ranked, *e.g.,* by digital hanging protocol system 108, as part of a digital hanging protocol associated with the clinical task being performed. Likewise, if medical personnel's 106 gaze only passes over a particular view briefly before they move on to another view of the medical imagery data, that may be a signal that the particular view is relatively unimportant.

Abnormality detection module 118 may be configured to detect abnormalities such as lesions on organs and/or tissues depicted in medical imagery. Abnormality detection module 118 may employ a variety of different techniques to detect lesions/abnormalities, such as object detection/recognition, edge detection, and so forth. In some embodiments, abnormality detection module 118 may employ one or more trained machine learning models, such as a convolutional neural network ("CNN"), to detect lesions in medical imagery. Although abnormality detection module 118 is depicted as part of medical examination software application 104, this is not meant to be limiting. In some embodiments, abnormality detection module 118 may be implemented in whole or in part as part of digital hanging protocol system 108.

Alternatively, in some embodiments, medical examination software application 104 itself may be implemented across multiple computing systems, *e.g.,* as a client portion and a server portion. In some such implementations, the server portion may be implemented on one more computing systems, sometimes referred to as "the cloud," which include greater and/or more powerful computing resources (*e.g.,* memory, processor cycles, battery power, etc.) than a client computing device executing the client portion of medical examination software application 104.

Among other things, lesions detected by abnormality detection module 118 may be used, *e.g.,* by digital hanging protocol system 108, to provide context for using one digital hanging protocol versus another. Suppose a particular sequence of views of medical imagery data of a subject is manually created, *e.g.,* by a radiologist, at the same time that a particular "reference" lesion is detected in the medical imagery data. In the future, when a similar lesion is detected, *e.g.,* by abnormality detection module 118, the digital hanging protocol used previously for the reference lesion may be selected for use once again and/or promoted over less contextually-relevant digital hanging protocols.

Organ localization module 120 may be configured to analyze the medical imagery data to establish a frame of reference with respect to one or more anatomical features of the subject depicted in the medical imagery data. For example, organ localization module 120 may use various techniques to identify and/or segment, in the medical imagery data, various anatomical features or other landmarks relative to each other. In some embodiments, abnormality detection module 118 may operate downstream from organ localization module 120, i.e., the lesion detection performed by abnormality detection module 118 may be informed by organs identified/located by organ localization module 120.

In some embodiments, organ localization module 120 may employ one or more trained machine learning models to segment medical imagery data into distinct organs, tissues, and/or lesions (if present). These machine learning models may take various forms, such as CNNs, and in some case may generate output that includes, for instance, pixel-wise and/or voxel-wise classifications of medical imagery data into specific organ "buckets" or "bins." In some embodiments, organ localization module 120 may also be configured to compute organ segmentations and register those segmentations with an anatomical atlas. In some embodiments, model-based segmentation may be employed to segment medical imagery data into distinct anatomical features. Although organ localization module 120 is depicted as part of medical examination software application 104, this is not meant to be limiting. In some embodiments, organ localization module 120 may be implemented in whole or in part as part of digital hanging protocol system 108.

Digital hanging protocol system 108 may be configured to learn, create, improve, and/or manage digital hanging protocols associated with a variety of clinical tasks. To this end, in some embodiments, digital hanging protocol system 108 may include and/or be operably coupled with a database 122 that stores, among other things, digital hanging protocols associated with clinical tasks. In some embodiments, digital hanging protocol system 108 (and/or HIS 102) may take the form of one or more computing systems, such as blade servers, that are operably coupled via one or more computer networks (not depicted) and that are configured to collectively and/or cooperatively perform selected aspects of the present disclosure. In some instances, digital hanging protocol system 108 and/or HIS 102 may be implemented in whole or in part on the cloud.

Digital hanging protocol system 108 may include a variety of different components that may perform selected aspects of the present disclosure. These components may be implemented using any combination of hardware and software. For example, in some implementations, digital hanging protocol system 108 may include a UI interaction engine 124 that is configured to cooperate with (*e.g.,* receive user interaction data from) UI module 112. In some embodiments, UI interaction engine 124 may be configured to receive a plurality of user input sequences from UI module 112.

These plurality of sequences may have been provided by medical personnel 106 at medical examination software application 104 in order to define/select a corresponding plurality of views of medical imagery data. Put another way, a sequence of user inputs may comprise application commands provided by medical personnel 106 to manipulate presentation of medical imagery data to form a desired view. The desired view may focus medical examination software application 104 on a respective portion of medical imagery data desired by medical personnel 106 to perform all or a portion of a respective clinical task. For example, one view may depict a lower anterior portion of an organ depicted in medical imagery data. Another view may depict an upper posterior portion of the organ. And so on. Generally speaking, these user input sequences/application commands may include panning, scrolling, rotating, and/or zooming, as well as other commands such as setting up look up tables, adjusting window sizes/orientations, and so forth.

In some embodiments, pauses between user inputs may also be logged by UI interaction engine 124. These pauses may correspond to a variety of different events, such as time intervals that medical personnel 106 spend looking at or reading particular views of medical imagery data, time intervals that are spent awaiting downloads of data (which may pose a latency concern), time spent awaiting computation of data (which can overwhelm memory and/or processing resources, and also may pose a latency concern), and so forth. In some embodiments, these learned pauses may be used by digital hanging protocol system 108 to perform various actions subsequently, such as proactively selecting and downloading, to medical examination software application 104, portions of medical imagery data that are likely to be heavily processed and/or demand substantial computing resources.

In some embodiments, digital hanging protocol system 108 may include a standards engine 126. Standards engine 126 may be configured to work in conjunction with other modules, such as UI interaction engine 124, to inform which new digital hanging protocols and/or views thereof should be learned by digital hanging protocol system 108 using techniques described herein, and/or which existing digital hanging protocols and/or views should be improved using techniques described herein. In particular, standards engine 126 may interface with one or more databases or depositories (not depicted) of medical standards, such as "best practices" or "standards of care," that include rules and/or guidelines under which medical personnel are required or suggested to operate.

For example, to examine a subject for a particular type of cancer, a standard of care may dictate that a clinician view at least *n* different views (*n* being a positive integer) of medical imagery data in order to properly support a positive or negative diagnosis. Standards engine 126 may impose and/or enforce this standard of care in a digital hanging protocol that is learned using techniques described herein. As another example, for a trauma patient for which medical imagery data has been acquired, medical personnel 106 may for first read portion(s) of the medical imagery data for organ ruptures. Next, medical personnel 106 may read portion(s) of the medical imagery data for bone fractures. Lastly, medical personnel 106 may read portion(s) of the medical imagery data for incidental damages.

Suppose a new standard of care is promulgated for a particular clinical task, and the digital hanging protocols in database 122 for that clinical task do not take this new standard into account. Standards engine 126 may attempt to update these digital hanging protocols to comport with the new standard of care, *e.g.,* by modifying existing predefined views associated with the digital hanging protocols and/or adding new view(s) to the digital hanging protocols.

In some embodiments, digital hanging protocol system 108 may include an image feature engine 128. Image feature engine 128 may be configured to cooperate with, e.g., receive data indicative of image features from, components of medical examination software application 104 such as settings module 114, abnormality detection module 118, and/or organ localization module 120. Image feature engine 128 may use this data to create associations between various image features and various digital hanging protocols and/or views thereof.

For example, image feature engine 128 may create an association between a given digital hanging protocol and one or more lesions detected in medical imagery data on which the given digital hanging protocol operated on historically. For example, whenever a particular tumor is detected, *e.g.,* by abnormality detection module 118, on a particular organ of a subject, radiologists may tend to implement the same or similar views to examine the tumor. Those views may be learned as part of a hanging protocol that can be selected for implementation whenever the same type of tumor is detected on the same organ of a new subject.

Additionally or alternatively, in some embodiments, image feature engine 128 may create an association between a predefined view of a digital hanging protocol and one or more lesions detected in medical imagery that is depicted in the predefined view. Suppose that, as part of a clinical task of performing routine prostate examination on a subject, a particular digital hanging protocol is selected and implemented. Suppose further that processing of medical imagery by abnormality detection module 118 also reveals a potentially cancerous growth on a particular region of the subject's prostate.

If that abnormality weren't detected, a view of that particular region of the subject's prostate may be presented no differently than other views. However, in light of the detected abnormality, the view of that particular region of the prostate may be promoted (*e.g.,* ranked higher than other views) so that it is surfaced (*e.g.,* rendered on a display, flagged, provided as a pop-up window) to medical personnel 106 more quickly and/or prominently than other views. In some cases, the view of the particularly problematic region may also be enhanced, *e.g.,* based on settings and other user interactions learned from similar clinical tasks performed in the past when similar abnormalities were detected.

In some embodiments, digital hanging protocol system 108 may include a user interest engine 130. User interest engine 130 may be configured to analyze various signals and/or data, such as data provided by modules 112-116, to gauge measures of interest by medical personnel 106 in particular predefined views relative to other predefined views. For example, user interest engine 130 may analyze data provided by UI module 112 to determine a measure of user interaction with one or more GUIs associated with one or more views. This information may include user interface actions taken by medical personnel 106, such as swiping, typing, clicking, dragging, and so forth. It also may include time spent by medical personnel 106 engaged with a particular view, regardless of explicit inputs received from medical personnel 106 during that time. The more medical personnel 106 interacts with, or spends time viewing, a particular view (or views sharing similar features), the more likely that view (or views sharing similar features) has a relatively large level of significance relative to other views that received less attention. In some embodiments, medical personnel 106 may explicitly or implicitly tag or otherwise save a particular view as one that should be incorporated into a digital hanging protocol. For example, if medical personnel 106 saves one or more key images for inclusion in a medical report, those views may also be preserved as part of a digital hanging protocol.

Similarly, settings module 114 may provide data about settings imposed by medical personnel 106 when performing clinical tasks, such as selected image features (*e.g.,* color balance, hue, saturation, intensity, filters applied, etc.), zoom settings, viewing angles, and so forth. Gaze detection module 116 also may provide data about gazes of medical personnel 106, which may be a direct indication of their interest in particular views. For example, in some embodiments, measures of user interest determined by user interest engine 130 may be determined at least in part based on respective time intervals of detected user gazes at particular views. One view (or a cluster of similar views) that receives a greater amount of gaze time may be assigned a greater measure of user interest than another view (or cluster of similar views) that received less gaze time from medical personnel 106.

Engines 124-130 are primarily concerned with learning new digital hanging protocols and/or views thereof, and improving existing digital hanging protocols. However, digital hanging protocol system 108 may also use these learned skills to help medical personnel 106 select digital hanging protocols and/or views to employ for particular clinical tasks and/or in particular contexts. To this end, in some implementations, digital hanging protocol system 108 may include a selection engine 132 that is configured to select digital hanging protocols and/or views thereof from database in response to various signals and/or data. Data indicative of the selected digital hanging protocols and/or views may then be provided to medical examination software application 104. Medical examination software application may prompt medical personnel 106 with the selected digital hanging protocols/views and/or may cause them to be implemented automatically.

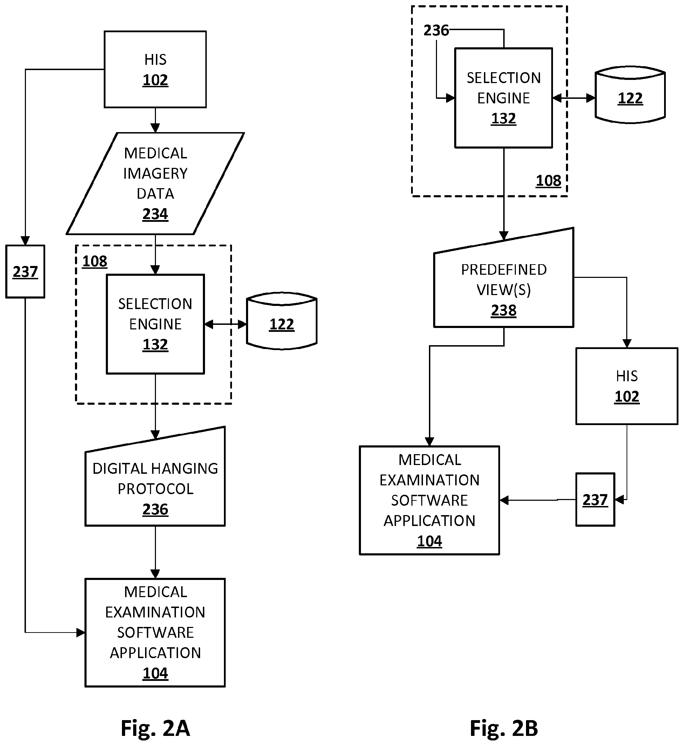

In some implementations, selection engine 132 may employ one or more trained machine learning models to select digital hanging protocols and/or views thereof to be implemented by medical examination software application 104. As one non-limiting example, and referring to Fig. 2A, in some implementations, HIS 102 may provide medical imagery data 234 (*e.g.,* CT scans, MRI data, ultrasound data, X-rays, etc.) to selection engine 132 of digital hanging protocol system 108. Selection engine 132 may apply all or parts of medical imagery data 234, such as various features extracted from medical imagery data 234 (*e.g.,* detected abnormalities, color histograms, patient data points, etc.), as input across one or more training machine learning models stored in database 122 to generate output. This output may indicate which digital hanging protocol 236 of a plurality of digital hanging protocols in database 122 should be selected for implementation by medical examination software application 104, as illustrated in Fig. 2A. The selected digital hanging protocol 236 and/or data indicative thereof may then be provided to medical examination software application 104.

In some embodiments, the selected hanging protocol 236 may also trigger medical examination software application 104 or another component to proactively download and/or cache selected portions of medical imagery data from HIS 102 that correspond to views of selected hanging protocol 236. This may alleviate latency problems associated with bandwidth limitations between remotely-working medical personnel 106 (and more particularly, medical examination software application 104 that they operate) and HIS 102. An example of this is indicated by the arrow at far left in Fig. 2A, which demonstrates how medical examination software application 104 may selectively and proactively download, from HIS 102 (or the underlying data sources 110), selected portions 237 of medical imagery data 234 that are required for rendering one or more views of selected digital hanging protocol 236.

Proactively prefetching selected portions 237 of medical imagery data 234 may also allow medical examination software application 104 to proactively execute various complex image processing algorithms on those proactively downloaded portions 237 of medical imagery data 234. By beginning this processing proactively, medical personnel 106 operating medical examination software application 104 may be presented with results of various image processing techniques, such as identification of abnormalities in selected portions 237 of medical imagery data 234, more quickly than if medical personnel 106 were required to manually initiate these image processing techniques, or if the image processing techniques were not initiated until all of medical imagery data 234 were downloaded to memory local to medical examination software application 104.

Fig. 2B depicts a similar data flow as Fig. 2A, except that as a result of the data flow of Fig. 2B, one or more views of a previously-selected digital hanging protocol 236 are selected. In particular, selection engine 132 starts by selecting the digital hanging protocol 236. This selection may be automatic, *e.g.,* based on output of a machine learning model as described with respect to Fig. 2A or based on heuristics, or manual, *e.g.,* based on explicit selection of digital hanging protocol 236 by medical personnel 106.

As described herein, selected digital hanging protocol 236 may include one or more predefined views of corresponding portions of medical imagery data 234. Selection engine 132 may apply all of medical imagery data, or selected portions of medical imagery data (*e.g.,* 237), as input across one or more trained machine learning models from database 122 to generate additional output. This additional output may be used to determine how to surface one or one more predefined view(s) 238 of the selected hanging protocol 236 to medical personnel 106. For example, the output may be used to rank the predefined views 238 (*e.g.,* by measures of user interest or relevance) in a list that is presented to medical personnel 106. Or, the output may be used to determine an order at which the predefined views 238 are automatically output, one after another (*e.g.,* as an animation), to medical personnel 106. In some embodiments, predefined views 238 (or their order or rankings) may also be provided to HIS 102, so that HIS 102 can proactively download selected portions 237 of medical imagery data 234 to medical examination software application 104. This further alleviates the bandwidth and latency concerns described previously.

Various types of machine learning models may be trained and used, *e.g.,* by selection engine 132, as described with respect to Figs. 2A and 2B. In some examples, the machine learning model may take the form of a support vector machine, a random forest, a decision tree, various types of neural networks such as a convolutional neural network ("CNN"), a recurrent neural network, an LSTM network, a GRU network, multiple machine learning models incorporated into an ensemble model, and so forth. In some examples, a machine learning model may learn weights in a training stage utilizing various machine learning techniques as appropriate to the classification task, which may include, for example linear regression, logistic regression, linear discriminant analysis, principal component analysis, classification trees, naive Bayes, k-nearest neighbors, learning vector quantization, support vector machines, bagging forests, random forests, boosting, AdaBoost, etc.

In examples described thus far, digital hanging protocol system 108 has learned from patient-agnostic views implemented by medical personnel 106 using medical examination software application 104. However, this is not meant to be limiting. In some embodiments, a patient's previous medical imagery-and in particular, lesions or other features previously located in that prior medical imagery-may be leveraged to implement a digital hanging protocol that is custom to a particular patient. For example, suppose patient John Doe previously had medical imagery taken of his prostate, and at least some of that prior medical imagery data was tagged or otherwise flagged as including one or more lesions (*e.g.,* polyps, tumors, etc.). When subsequent medical imagery data is acquired from John Doe to reevaluate those lesion(s), the same or similar views that best captured the lesions previously (including capturing their sizes, extents, associated vessel diameters, etc.) may be implemented again. Accordingly, medical personnel 106 can get a true comparison of relevant portion(s) of John Doe's medical imagery data over time. In some implementations, medical notes and/or other data (*e.g.,* stored with PACs data) may be parsed into structured reports. These structured reports can be an additional source of data that can be used to learn a custom digital hanging protocol for a particular patient, *e.g.,* by virtue of these notes identifying particular locations and/or anatomical regions that included lesions in the past.

While examples described herein generally relate to healthcare, this is not meant to be limiting. Techniques described herein may be applicable in any scenario in which portions of imagery data are viewed in similar manners by multiple entities.

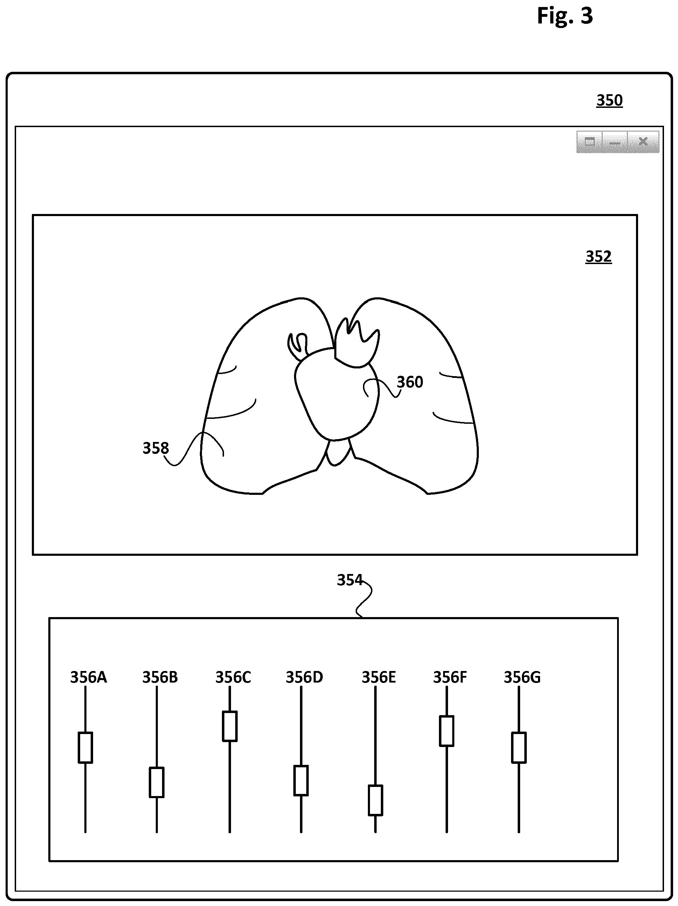

Fig. 3 depicts an example GUI 350 that may be rendered by medical examination software application 104 to allow medical personnel 106 to read medical imagery, in accordance with various embodiments. GUI 350 includes a first window 352 for displaying a view of some anatomical features of a subject. In this example, the depicted anatomical features include lungs 358 and a heart 360 of the subject. GUI 350 also includes a second window 354 that contains a plurality of operable elements 356A-G that are operable by medical personnel 106 to alter various settings associated with the view implemented in first window 352. For example, operable elements 356A-G in Fig. 3 take the form of sliders that can be adjusted by medical personnel 106 to, for instance, adjust various images settings such as color balance, gamma levels, saturation, hue, brightness, contrast, etc.

As described previously, manipulation of elements such as operable elements 356A-G, as well as other controls such as panning, zooming, tilting, and/or rotating the view captured in first window 352, may be captured, *e.g.,* by UI interaction engine 124. These user interactions may then be used, *e.g.,* by digital hanging protocol system 108, to learn a new digital hanging protocol, or to improve one or more views of an existing digital hanging protocol. Other phenomena that may be logged and used by digital hanging protocol system 108 include but are not limited to times spent viewing the view in first window 352 with particular settings in place, times the user's gaze was detected looking at these views with particular settings in place, detection lesions, and so forth.

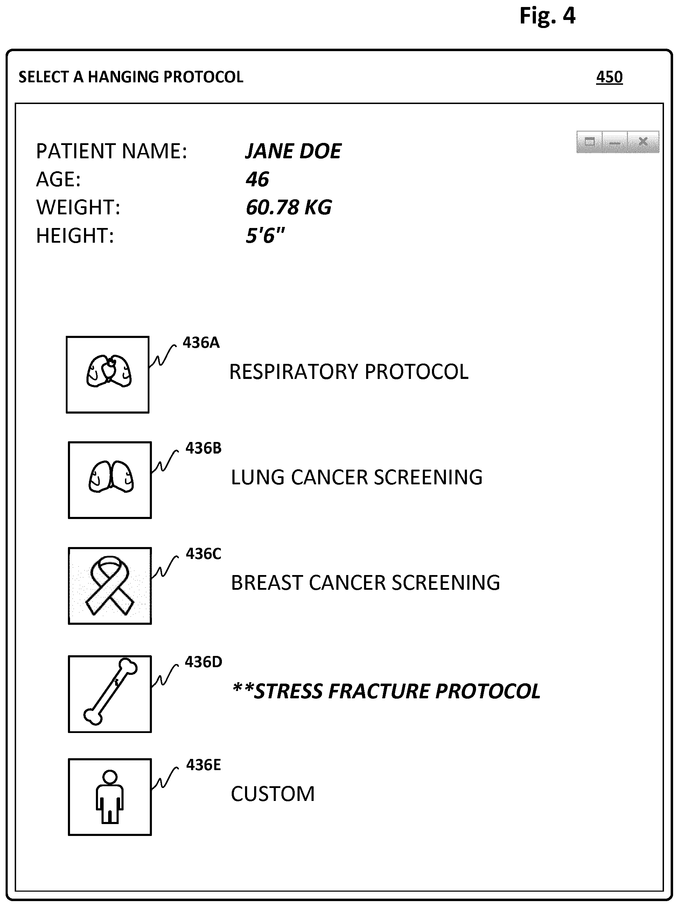

Fig. 4 depicts another example GUI 450 that may be rendered, *e.g.,* by medical examination software application 104, to allow medical personnel 106 to select a digital hanging protocol to implement for a particular subject named "Jane Doe." Medical personnel 106 may be presented GUI 450 when, for instance, they receive notification that medical imagery for Jane Doe is available, *e.g.,* at HIS 102. A list of selectable elements corresponding to candidate digital hanging protocols 436A-E are provided. Medical personnel 106 may select one of these candidate digital hanging protocols 436A-E to be implemented by medical examination software application 104. In some embodiments, this selection may cause one or more portions of the medical imagery to be proactively downloaded, *e.g.,* by medical examination software application 104 from HIS 102 or the underlying data sources 110. These portions that are selectively downloaded may correspond to predefined views associated with the selected digital hanging protocol.

In Fig. 4, candidate digital hanging protocols 436A-E are presented (i.e. are ranked) in a particular order. This order/ranking may be determined, *e.g.,* by medical examination software application 104 or by digital hanging protocol system 108, based on a variety of features on which the candidate digital hanging protocols 436A-E may be indexed. In some embodiments, candidate digital hanging protocols 436A-E may be indexed and/or ranked based on measures of user interest associated with them and/or their underlying predefined views. For example, more popular and/or more frequently used digital hanging protocols may be promoted over and/or presented ahead of less popular/frequently used digital hanging protocols.

Additionally or alternatively, in some embodiments, candidate digital hanging protocols 436A-E may be presented in an order that is determined based on processing of medical imagery data using machine learning model(s), similar to Figs. 2A-B. For example, all or portions of Jane Doe's medical imagery data may be applied as input across a machine learning model to generate a semantically-rich embedding in latent space. Distances in the latent space (*e.g.,* Euclidian distance, cosine similarity, dot product, etc.) between this semantically-rich embedding and other "reference" embeddings generated based on medical imagery acquired from prior patients may be determined. Digital hanging protocol(s) that were used previously in association with the closest neighbor patient(s) in the latent space may then be identified. These identified digital hanging protocol(s) may be used for purposes of selecting and/or ranking candidate digital hanging protocols that are presented as part of GUI 450 as options for examining Jane Doe's health.

For example, suppose Jane Doe's medical imagery included an X-ray of a bone in which stress fracture is detected, *e.g.,* by abnormality detection module 118. Alternatively, suppose Jane Doe complained to her physician of pain that the physician noted as a possible stress fracture. In either case, candidate digital hanging protocol 436D ("Stress Fracture Protocol") is visually emphasized (*e.g.,* with "**") to inform medical personnel 106 that this candidate digital hanging protocol is worthy of further inquiry. If the stress fracture's specific location in Jane Doe is identified (automatically by abnormality detection module 118 or by the physician), then predefined views of stress fracture protocol 436D that depict that location may be promoted over other predefined views. In some embodiments, in addition to or instead of visually emphasizing candidate digital hanging protocol 436D, candidate digital hanging protocols 436A-E may be reordered so that digital hanging protocol 436D appears at the top of the list.

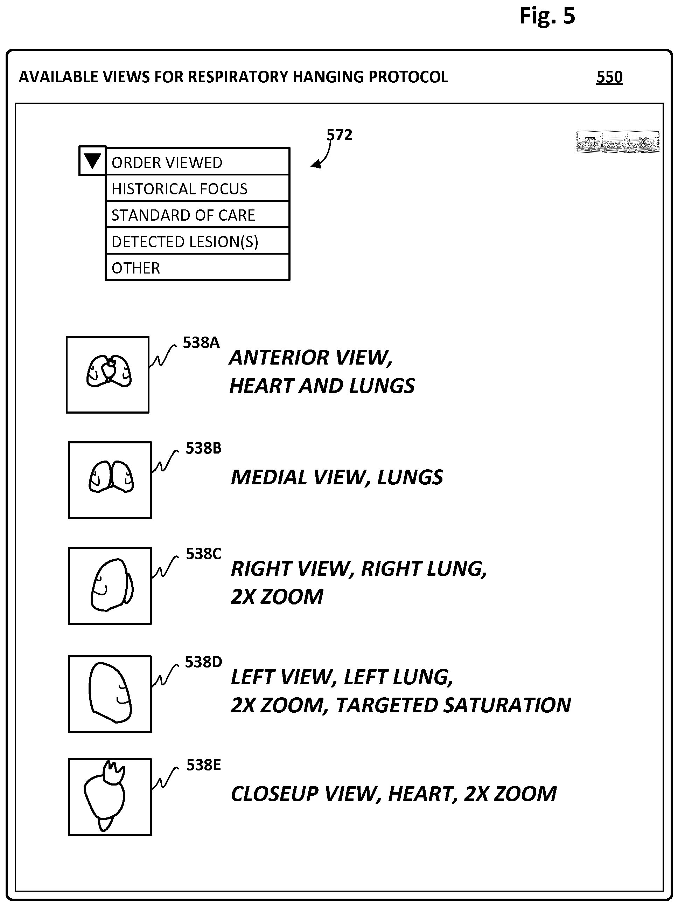

Fig. 5 depicts another GUI 550 that may be rendered once a particular digital hanging protocol-"Respiratory Hanging Protocol-is selected (automatically or manually by medical personnel 106) for implementation by medical examination software application 104. In Fig. 5, a plurality of predefined views 538A-E are depicted as selectable graphical elements. In this example, each selectable element includes, on the left, a thumbnail showing a preview of the view that will be implemented if that graphical element is selected.

Views 538A-E may be ordered/ranked based on various signals. In some embodiments, they may be ranked by popularity and/or frequency of use, *e.g.,* when the Respiratory Hanging Protocol is implemented. Additionally or alternatively, in some embodiments, they may be ranked by measures of user interest, which may indicate how long medical personnel 106 previously lingered on each view in the aggregate (*e.g.,* as determined by UI interaction engine 124, UI module 112, gaze detection module 116, and/or user interest engine 130).

A drop-down list 572 is also provided that allows medical personnel 106 to reorder the views 538A-E based on various indices. These indices may include but are not limited to the most common order in which the views are implemented by medical personnel 106 generally, historical focus (*e.g.,* user interest described previously), standard of care (*e.g.,* an order/ranking that is dictated by an established rule, law, regulation, court ruling, etc.), whether suspected lesions have been detected in the medical imagery data that is depicted in each view (views that will depict suspected lesions may be promoted over other views), and so forth.

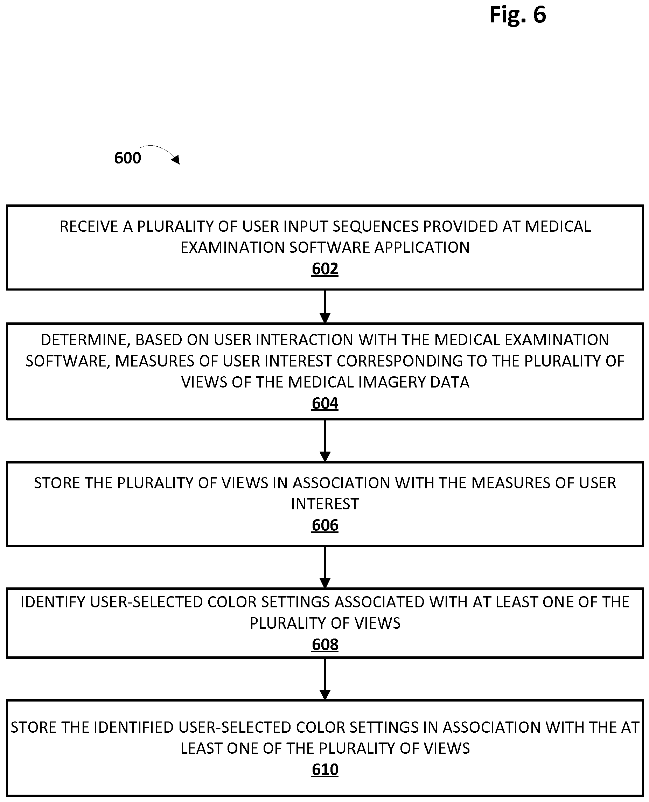

Fig. 6 illustrates a flowchart of an example method 600 for implementing a "learning phase" in which medical personnel interactions with medical examination software application 104 are examined to learn new digital hanging protocols and/or views thereof. This learning phase may be implemented prior to an "application phase" in which digital hanging protocol system 108 applies what it has learned to help medical personnel 106 select and/or automatically implement digital hanging protocols and/or views thereof. The learning phase may also continue to be implemented in parallel with application-in order words, digital hanging protocol system 108 may continue to refine its knowledge based on user interaction with medical examination software application 104.

The operations of Fig. 6 can be performed by various components described herein, such as medical examination software application 104 and/or digital hanging protocol system 108. For convenience, operations of method 600 will be described as being performed by a system configured with selected aspects of the present disclosure. Other implementations may include additional operations than those illustrated in Fig. 6, may perform step(s) of Fig. 6 in a different order and/or in parallel, and/or may omit one or more of the operations of Fig. 6.

At block 602, the system may receive a plurality of user input sequences provided at medical examination software application 104. As described herein, medical examination software application facilitates engagement by medical personnel 106 with medical imagery data, *e.g.,* to make diagnosis, formulate treatment plans, plan surgeries, etc. The plurality of user input sequences may effectively define a corresponding plurality of views of the medical imagery data. For example, one user input sequence may include application commands provided by medical personnel 106 to pan, zoom, and/or rotate medical imagery data so that medical examination software application 104 is focused on a respective portion of the medical imagery data that, *e.g.,* enables medical personnel to make a diagnosis. Put another way, at block 602, the system collects information about how medical personnel selects and/or defines a plurality of views of medical imagery data, so that this information can be used later under similar circumstances.

At block 604, the system, *e.g.,* by way of user interest engine 130, may determine, based on user interaction with the medical examination software, measures of user interest corresponding to the plurality of views of the medical imagery data. For example, UI module 112 and/or UI interaction engine 124 may detect how many commands a user issued to configure each view (more commands may mean the user was more interested in that view, or the ultimate view that resulted), and may base the measures of user interest on these commands. Additionally or alternatively, user interest engine 130 may utilize gaze data provided by gaze detection module 116 to determine how long medical personnel linger on each view, and therefore, which views are likely of greater interest.

At block 606, the system may store, *e.g.,* in database 122, the plurality of views in association with the measures of user interest determined at block 604. These measures of interest may be used later as described herein to select which views are presented to medical personnel, and/or to rank or order a list of views that are presented to medical personnel 106 (as shown in Fig. 2B).

At optional block 608, the system, *e.g.,* by way of settings module 114 and/or image feature engine 128, may identify user-selected color settings associated with at least one of the plurality of views. For example, after medical personnel 106 manipulates image settings such as color levels, gamma levels, hue, saturation, contrast, etc., in order to achieve a clear view of a lesion in a portion of medical imagery data, these settings (*e.g.,* color histogram, other settings) may be identified. At optional block 610, the system may store the identified user-selected color settings in association with the at least one of the plurality of views.

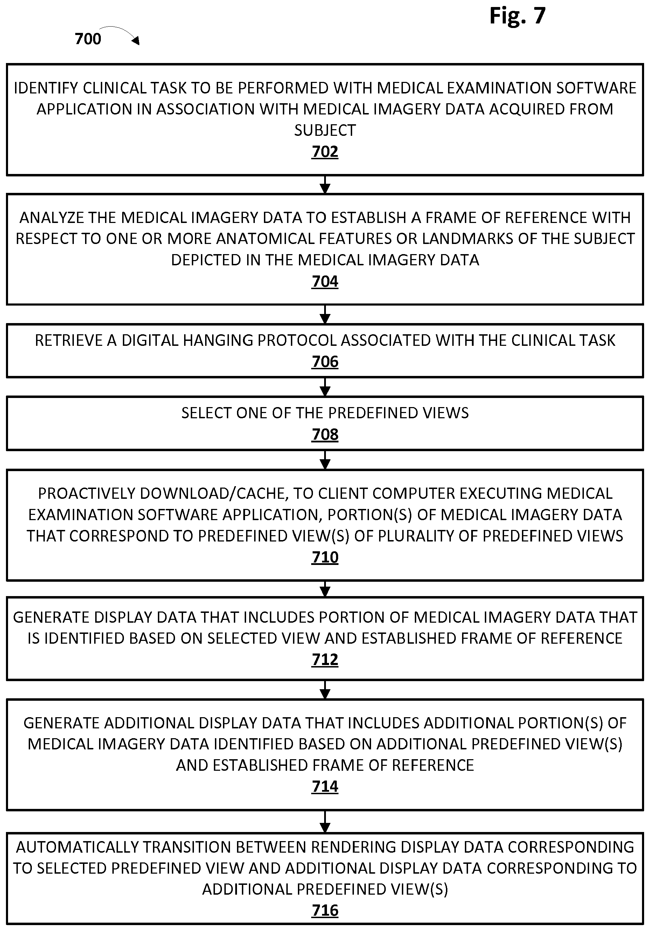

Fig. 7 illustrates a flowchart of an example method 700 for implementing an "application phase" in which information about digital hanging protocols learned as part of the process 600 of Fig. 6 may be applied to facilitate more efficient and/or accurate reading of medical imagery data by medical personnel 106. The operations of Fig. 7 can be performed by various components described herein, such as medical examination software application 104 and/or digital hanging protocol system 108. For convenience, operations of method 700 will be described as being performed by a system configured with selected aspects of the present disclosure. Other implementations may include additional operations than those illustrated in Fig. 7, may perform step(s) of Fig. 7 in a different order and/or in parallel, and/or may omit one or more of the operations of Fig. 7.

At block 702, the system may identify a clinical task to be performed with medical examination software application 104 in association with medical imagery data acquired from a subject. For example, medical personnel 106 such as a radiologist may be referred a patient from another clinician such as a primary care or emergency room physician, *e.g.,* to perform a clinical task of evaluating a suspected trauma experienced by the patient. The referring clinician or the radiologist may request that medical imagery be acquired from the patient. The radiologist may launch medical examination software application 104 to read the medical imagery data acquired from the patient. In some embodiments, the radiologist may input the clinical task to be performed.

At block 704, the system, *e.g.,* by way of organ localization module 120 and/or image feature engine 128, may analyze the medical imagery data to establish a frame of reference with respect to one or more anatomical features or landmarks of the subject depicted in the medical imagery data. For example, the system may perform segmentation analysis as described previously to classify pixels or voxels of medical imagery data as belonging to particular anatomical features. A particular landmark (*e.g.,* the heart, a particular blood vessel, a particular bone or joint, the pancreas, etc.) may then be used to establish a frame of reference relative to which predefined view(s) from a digital hanging protocol can be implemented.

For example, suppose a predefined view of a digital hanging protocol depicts gall bladders from a particular perspective. At block 704, the current medical imagery data (*e.g.,* the data currently being read by the radiologist) may be analyzed to locate the current patient's gall bladder. This identified landmark can then be used as the frame of reference to recreate a similar view of the current patient's gall bladder. In particular, one or more views of the current patient's gall bladder may be generated and/or adjusted, *e.g.,* by panning, zooming, tilting, rotating, translating, etc., until a similar perspective of the current patient's gall bladder is achieved.

At block 706, the system may retrieve, *e.g.,* from database 122, a digital hanging protocol associated with the clinical task. For example, based on the clinical task identified at block 702, medical examination software application 104 may present one or more candidate digital hanging protocols from which the radiologist may select, as depicted in Fig. 4. The radiologist may then select a particular digital hanging protocol for implementation by medical examination software application 104.

As described herein, the digital hanging protocol may include a plurality of predefined views learned (*e.g.,* during method 600) from previous performances of the clinical task. In some embodiments, the plurality of predefined views may be indexed by corresponding measures of user interest, which as explained previously may be based on historical user interaction with medical examination software application 104. In some such embodiments, the historical user interaction with medical examination software application 104 may include time intervals spent by users interacting with the plurality of predefined views.

At block 708, the system may select one of the predefined views. This selection may be performed manually (*e.g.,* by the radiologists) or automatically based on various factors (*e.g.,* measures of user interest, detected lesions, etc.). For example, and as noted previously, in some embodiments, abnormality detection module 118 may analyze the medical imagery data to detect one or more lesions of the subject. In some such embodiments, the selection of block 708 may include selecting one of the predefined views based on the detected one or more lesions. Additionally or alternatively, in some embodiments, the digital hanging protocol retrieved at block 702 may be selected from a library of digital hanging protocols based on the detected one or more lesions.

In some embodiments, the medical imagery data acquired from the current patient may be stored on a server computer that is remote from a client computer that executes medical examination software application 104. In some such embodiments, at block 710, the system may proactively download and/or cache, *e.g.,* to the client computer that executes medical examination software application 104, one or more portions of the medical imagery data that correspond to one or more predefined views (selected or unselected) of the plurality of predefined views. The operations of block 710 may occur in series with, or in parallel to, any other operation of Fig. 7.

At block 712, the system may generate, for rendition on a display, display data that includes a portion of the medical imagery data that is identified based on the selected view and the established frame of reference. For example, the established frame of reference may be used to translate, rotate, pan, zoom, or otherwise spatially arrange the medical imagery data so that medical personnel 106 obtains a view that is preconfigured to depict anatomical features they intend to study based on the clinical task they are performing.

In some embodiments, at block 714, the system may generate, for rendition on the display, additional display data that includes one or more additional portions of the medical imagery data that are identified based on one or more additional predefined views of the selected digital hanging protocol and the established frame of reference. At block 716, the system may automatically transition between rendering the display data corresponding to the selected predefined view and the additional display data corresponding to the one or more additional predefined views. For example, the radiologist may be presented with an animated and/or curated presentation that transitions between the various views of the digital hanging protocol automatically, without the radiologist having to provide input to trigger the transitions.

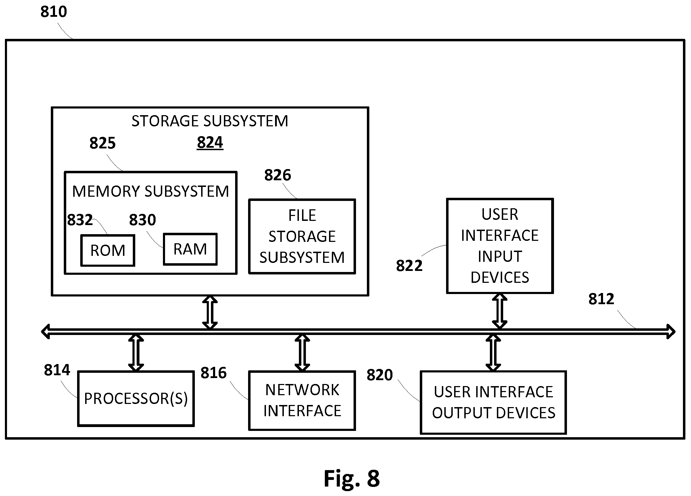

Fig. 8 is a block diagram of an example computing device 810 that may optionally be utilized to perform one or more aspects of techniques described herein. Computing device 810 typically includes at least one processor 814 which communicates with a number of peripheral devices via bus subsystem 812. These peripheral devices may include a storage subsystem 824, including, for example, a memory subsystem 825 and a file storage subsystem 826, user interface output devices 820, user interface input devices 822, and a network interface subsystem 816. The input and output devices allow user interaction with computing device 810. Network interface subsystem 816 provides an interface to outside networks and is coupled to corresponding interface devices in other computing devices.

User interface input devices 822 may include a keyboard, pointing devices such as a mouse, trackball, touchpad, or graphics tablet, a scanner, a touchscreen incorporated into the display, audio input devices such as voice recognition systems, microphones, and/or other types of input devices. In some implementations in which computing device 810 takes the form of a head-mounted display or smart glasses, a pose of a user's eyes may be tracked for use, *e.g.,* alone or in combination with other stimuli (*e.g.,* blinking, pressing a button, etc.), as user input. In general, use of the term "input device" is intended to include all possible types of devices and ways to input information into computing device 810 or onto a communication network.

User interface output devices 820 may include a display subsystem, a printer, a fax machine, or non-visual displays such as audio output devices. The display subsystem may include a cathode ray tube (CRT), a flat-panel device such as a liquid crystal display (LCD), a projection device, one or more displays forming part of a head-mounted display, or some other mechanism for creating a visible image. The display subsystem may also provide non-visual display such as via audio output devices. In general, use of the term "output device" is intended to include all possible types of devices and ways to output information from computing device 810 to the user or to another machine or computing device.

Storage subsystem 824 stores programming and data constructs that provide the functionality of some or all of the modules described herein. For example, the storage subsystem 824 may include the logic to perform selected aspects of methods 600 and 700 described herein, as well as to implement various components depicted in Figs. 1-2B.

These software modules are generally executed by processor 814 alone or in combination with other processors. Memory 825 used in the storage subsystem 824 can include a number of memories including a main random access memory (RAM) 830 for storage of instructions and data during program execution and a read only memory (ROM) 832 in which fixed instructions are stored. A file storage subsystem 826 can provide persistent storage for program and data files, and may include a hard disk drive, a floppy disk drive along with associated removable media, a CD-ROM drive, an optical drive, or removable media cartridges. The modules implementing the functionality of certain implementations may be stored by file storage subsystem 826 in the storage subsystem 824, or in other machines accessible by the processor(s) 814.

Bus subsystem 812 provides a mechanism for letting the various components and subsystems of computing device 810 communicate with each other as intended. Although bus subsystem 812 is shown schematically as a single bus, alternative implementations of the bus subsystem may use multiple busses.

Computing device 810 can be of varying types including a workstation, server, computing cluster, blade server, server farm, or any other data processing system or computing device. Due to the ever-changing nature of computers and networks, the description of computing device 810 depicted in Fig. 8 is intended only as a specific example for purposes of illustrating some implementations. Many other configurations of computing device 810 are possible having more or fewer components than the computing device depicted in Fig. 8.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the operations or acts of the method is not necessarily limited to the order in which the operations or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope.

## Claims

1. A method implemented using one or more processors, comprising:
identifying (702) a clinical task to be performed with a medical examination software application (104) in association with medical imagery data acquired from a subject;
analyzing (704) the medical imagery data to establish a frame of reference with respect to one or more anatomical features of the subject depicted in the medical imagery data;
retrieving (706) a digital hanging protocol associated with the clinical task, wherein the digital hanging protocol includes a plurality of predefined views learned from previous performances of the clinical task;
selecting (708) one of the predefined views; and
generating (712), for rendition on a display, display data that includes a portion of the medical imagery data that is identified based on the selected view and the established frame of reference.

2. The method of claim 1, wherein the plurality of predefined views are indexed by corresponding measures of prior interest.

3. The method of claim 2, wherein the corresponding measures of prior interest are based on historical user interaction with the medical examination software application.

4. The method of claim 3, wherein the historical user interaction with the medical examination software application includes time intervals spent by users interacting with the plurality of predefined views.

5. The method of claim 1, further comprising analyzing the medical imagery data to detect one or more lesions of the subject.

6. The method of claim 5, wherein selecting one of the predefined views comprises selecting one of the predefined views based on the detected one or more lesions.

7. The method of claim 5, further comprising selecting the digital hanging protocol from a library of digital hanging protocols based on the detected one or more lesions.

8. The method of claim 1, wherein the medical imagery data is stored on a server computer and the medical examination software application executes at least in part on a client computer in network communication with the server computer.

9. The method of claim 8, further comprising proactively downloading and caching (710), by the client computer from the server computer, one or more portions of the medical imagery data that correspond to one or more unselected predefined views of the plurality of predefined views.

10. The method of claim 1, further comprising:
generating (714), for rendition on the display, additional display data that includes one or more additional portions of the medical imagery data that are identified based on one or more additional predefined views and the established frame of reference; and
automatically transitioning (716) between rendering the display data corresponding to the selected predefined view and the additional display data corresponding to the one or more additional predefined views.

11. A system comprising one or more processors and memory storing instructions that, in response to execution of the instructions by the one or more processors, cause the one or more processors to:
receive (602) a plurality of user input sequences provided at a medical examination software application (104),
wherein the medical examination software application facilitates user engagement with medical imagery data,
wherein the plurality of user input sequences define a corresponding plurality of views of the medical imagery data, and
wherein each of the plurality of views focuses the medical examination software application on a respective portion of the medical imagery data;
determine (604), based on user interaction with the medical examination software application, measures of user interest corresponding to the plurality of views of the medical imagery data; and
store (606), in a database (122), the plurality of views in association with the measures of user interest.

12. The system of claim 11, wherein the measures of user interest are determined at least in part based on respective time intervals of detected user gazes.

13. The system of claim 11, wherein the measures of user interest are determined at least in part based on respective time intervals spent by one or more users interacting with the plurality of views of the medical imagery data.

14. The system of claim 11, further comprising instructions to identify (608) user-selected color settings associated with at least one of the plurality of views, wherein the user-selected color settings are stored in association with the at least one of the plurality of views.

15. At least one non-transitory computer-readable medium comprising instructions that, in response to execution of the instructions by one or more processors, cause the one or more processors to perform the following operations:
identifying (702) a clinical task to be performed with a medical examination software application (104) in association with medical imagery data acquired from a subject;
analyzing (704) the medical imagery data to establish a frame of reference with respect to one or more anatomical features of the subject depicted in the medical imagery data;
retrieving (706) a digital hanging protocol associated with the clinical task, wherein the digital hanging protocol includes a plurality of predefined views learned from previous performances of the clinical task;
selecting (708) one of the predefined views; and
generating (712), for rendition on a display, display data that includes a portion of the medical imagery data that is identified based on the selected view and the established frame of reference.
